# EUROPEAN PATENT APPLICATION

(11) **EP 4 379 040 A1**
(43) Date of publication of application: **05.06.2024**
(21) Application number: 22861678.5
(22) Date of filing: 23.08.2022
(51) Int. Cl.: C12M 1/34, C12M 1/00, C12M 3/06, C12M 1/32

(54) **CELL CULTURE DEVICE**

(30) Priority: 25.08.2021 KR 20210112722
(71) Applicant: The Asan Foundation, Seoul 05505 (KR); University Of Ulsan Foundation For Industry Cooperation, Ulsan 44610 (KR)
(72) Inventor: JEONG, Gi Seok, Seoul 05505 (KR)
(74) Representative: AWA Sweden AB
(86) International application number: PCT/KR2022/012554
(87) International publication number: WO 2023/027469

(57) **Abstract**

The present disclosure relates to a cell culture device.

An aspect of the present disclosure may include a culture medium supply unit including a base, a first reservoir disposed on the base and having an accommodation space, which accommodates the culture medium and has one surface in which a through hole through which the culture medium is discharged is formed, and a culture medium control member disposed to pass through the through hole and configured to control a flow of the culture medium discharged from the first reservoir.

## Description

### TECHNICAL FIELD

The present disclosure relates to a cell culture device.

### BACKGROUND ART

A microenvironment that allows cells or the like to receive nutrients and release metabolites through blood flow is implemented in a human body. The cells in the human body may perform various metabolic activities and grow based on the microenvironment.

Accordingly, when culturing cultures such as three-dimensional cell spheroids or organoids for medical, biological, and engineering experiments, it is desirable to implement an environment similar to the microenvironment of the human body. For example, when implementing a culture environment that approximates such a microenvironment, cultures may be efficiently and uniformly cultured. In addition, even when evaluating drugs while providing various drugs to cultures, it is preferable to implement an environment similar to the microenvironment of the human body.

Meanwhile, even when an environment similar to a human microenvironment is created, in order to perform various comparative experiments on cultures, there is a need for a device capable of accommodating multiple cells and a device capable of supplying various types of drugs to the cells.

Accordingly, there is a need to develop a cell culture device capable of controlling a flow rate and a flow volume of a culture medium to implement an environment similar to the microenvironment of the human body, and furthermore the device capable of culturing a plurality of cells and supplying various types of drugs to the cells.

### DESCRIPTION OF EMBODIMENTS

### TECHNICAL PROBLEM

The present disclosure is directed to providing a device capable of efficiently performing culturing and drug evaluation of cultures such as three-dimensional cell spheroids or organoids, in large numbers and over long periods of time.

The present disclosure is also directed to providing a device capable of controlling a flow rate and a flow volume of a culture medium.

The present disclosure is also directed to providing a device capable of simultaneously culturing various cultures and simultaneously providing various drugs to the cultures.

However, these objects are exemplary, and the scope of the present disclosure is not limited thereto.

### TECHNICAL SOLUTION TO PROBLEM

In order to achieve the above-described object, an aspect of the present disclosure may include a culture medium supply unit including a base, a first reservoir disposed on the base and having an accommodation space, which accommodates the culture medium and has one surface in which a through hole through which the culture medium is discharged is formed, and a culture medium control member disposed to pass through the through hole and configured to control a flow of the culture medium discharged from the first reservoir.

### ADVANTAGEOUS EFFECTS OF DISCLOSURE

The present disclosure can provide a device capable of efficiently performing culturing of cultures such as three-dimensional cell spheroids or organoids in large numbers and over long periods of time, and drug evaluation using cancer organoids and cancer cell spheroids.

Further, the present disclosure can provide a device capable of controlling a flow rate and a flow volume of a culture medium.

Further, the present disclosure can provide a device capable of simultaneously culturing various cultures and simultaneously providing various drugs to the cultures.

However, these effects are exemplary and do not limit the present disclosure.

### BRIEF DESCRIPTION OF DRAWINGS

FIG. 1 is a view schematically illustrating a cell culture device according to an embodiment of the present disclosure.
FIG. 2 is a view for describing a state in which a cover is provided in a reservoir of FIG. 1.
FIG. 3 is a view schematically illustrating an embodiment of the reservoir of FIG. 1
FIG. 4 is a view for describing a method of controlling a flow of a culture medium.
FIG. 5 is a view for describing a usage example of the cell culture device according to an embodiment of the present disclosure.
FIG. 6 is a view schematically illustrating a plate unit of FIG. 1.
FIG. 7 is an enlarged view of portion A of FIG. 6.
FIG. 8 is a view schematically illustrating one modified example of the plate unit.
FIG. 9 is a view schematically illustrating another modified example of the plate unit.
FIG. 10 is a view for describing the flow of the culture medium.
FIG. 11 is a view for describing a process of supplying the culture medium to a culture.
FIG. 12 is a view for describing the principle in which the culture medium flows.

### BEST MODE

In order to achieve the above-described object, an aspect of the present disclosure may include a culture medium supply unit including a base, a first reservoir disposed on the base and having an accommodation space, which accommodates the culture medium and has one surface in which a through hole through which the culture medium is discharged is formed, and a culture medium control member disposed to pass through the through hole and configured to control a flow of the culture medium discharged from the first reservoir.

In some embodiments, the culture medium supply unit may further include a second reservoir disposed on the base to be spaced apart from the first reservoir.

In some embodiments, the reservoir may include at least one partition wall configured to partition the accommodation space into a plurality of spaces.

In some embodiments, in the reservoir, a through hole is formed in each of the plurality of spaces partitioned by the partition wall.

In some embodiments, the culture medium control member may be made of a porous material.

In some embodiments, the cell culture device may further include a plate unit including a channel configured to guide a path through which the culture medium is supplied from the culture medium supply unit, and at least one well disposed to be connected to the channel.

In some embodiments, the plate unit may be detachable from the culture medium supply unit.

In some embodiments, the channel may be provided in plurality.

In some embodiments, the well may be disposed in plurality along a length of the channel.

### MODE OF DISCLOSURE

While the present disclosure is susceptible to various modifications and alternative forms, specific embodiments thereof are shown by way of example in the drawings and will herein be described in detail. Advantages and features of the present disclosure and methods for accomplishing the same will be more clearly understood from embodiments described below with reference to the drawings. However, the present disclosure is not limited to the embodiments disclosed below but may be implemented in various forms.

Hereinafter, the embodiments of the present disclosure will be described in detail with reference to the accompanying drawings, but when describing with reference to the drawings, equal or corresponding components will be referred to as the same reference numerals, and redundant descriptions thereof will be omitted.

In the following embodiments, the terms "first," "second," and the like have been used to distinguish one component from another, rather than limitative in all aspects.

In the following embodiments, singular forms are intended to include plural forms as well, unless the context clearly indicates otherwise.

It will be further understood that the terms "including" and/or "having" used herein specify the presence of stated features or components, but do not preclude the presence or addition of one or more other features or components.

Sizes of components in the drawings may be exaggerated or reduced for convenience of description. For example, the size and thickness of each component shown in the drawings are arbitrarily represented for convenience of description, and thus, the present disclosure is not necessarily limited thereto.

In the following embodiments, an x-axis, a y-axis, and a z-axis are not limited to three axes of the rectangular coordinate system, and may be interpreted in a broader sense. For example, the x-axis, the y-axis, and the z-axis may be perpendicular to one another, or may represent different directions that are not perpendicular to one another.

When a certain embodiment may be implemented differently, a specific process order may be performed differently from the described order. For example, two consecutively described processes may be performed substantially at the same time or performed in an order opposite to the described order.

Hereinafter, based on the principle described above, a cell culture device 10 according to the present disclosure will be described with reference to the drawings.

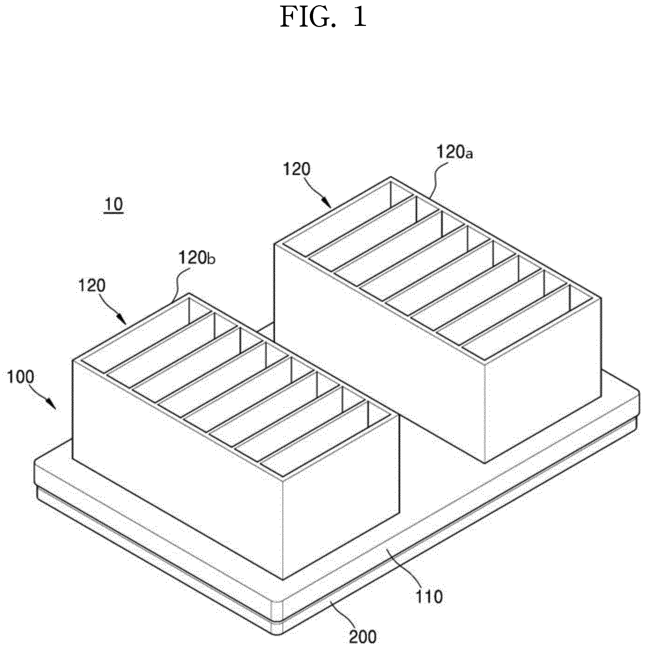

FIG. 1 is a view schematically illustrating the cell culture device 10 according to an embodiment of the present disclosure.

Referring to FIG. 1, the cell culture device 10 according to an embodiment of the present disclosure may include a culture medium supply unit 100 and a plate unit 200.

The culture medium supply unit 100 may be a means for receiving and storing a culture medium necessary for culturing cultures and supplying the stored culture medium to the plate unit 200.

The plate unit 200 may be a means for receiving the culture medium from the culture medium supply unit 100, providing a path through which the supplied culture medium flows, and providing a space in which the culture medium is supplied to the cultures. This will be described in detail later.

The cultures may include three-dimensional cells, and specifically, may be three-dimensional cell spheroids, organoids, and the like. For example, the cultures may be multi-cell cancer spheroids, organ cell spheroids, patient-derived cancer organoids, general organ organoids, or the like, but the present disclosure is not limited thereto.

The culture medium may refer to any material that contains nutrients or other components necessary for culturing cultures. In addition, the culture medium may include drugs supplied to the cultures for drug evaluation, and may include various drugs such as, for example, anti-cancer agents, promoters, and the like. However, the present disclosure is not limited thereto.

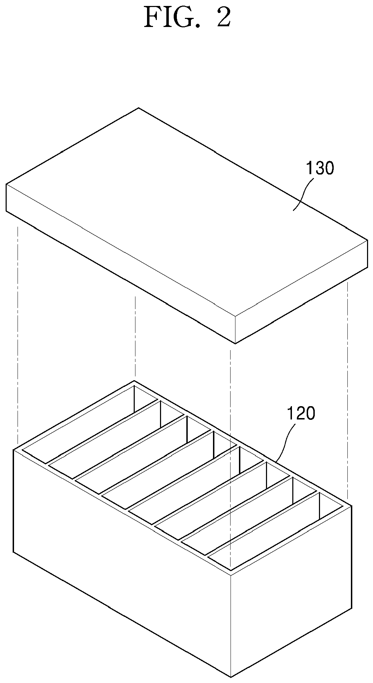

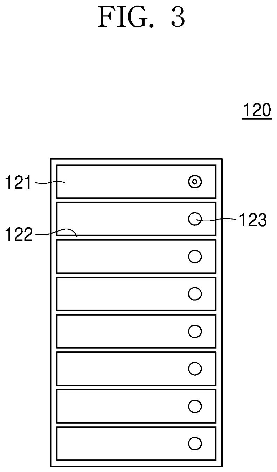

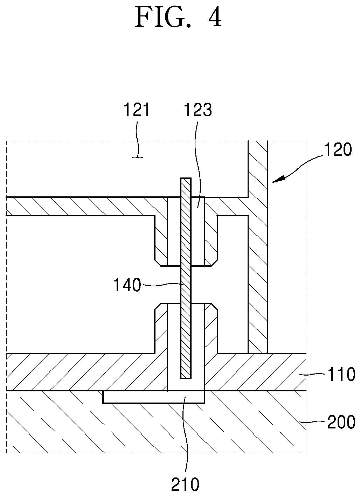

FIG. 2 is a view for describing a state in which a cover 130 is provided in a reservoir 120 of FIG. 1, FIG. 3 is a view schematically illustrating an embodiment of the reservoir 120 of FIG. 1, and FIG. 4 is a view for describing a method of controlling a flow of a culture medium.

Referring to FIGS. 1 to 4, the culture medium supply unit 100 according to an embodiment of the present disclosure may include a base 110, the reservoir 120 disposed on the base 110 and having an accommodation space 121, which accommodates the culture medium and has one surface in which a through hole 123 through which the culture medium is discharged is formed, and a culture medium control member 140 disposed to pass through the through hole 123 and configured to control the flow of the culture medium discharged from the reservoir 120.

The base 110 may form one surface of the culture medium supply unit 100 and support the base 110. In an embodiment, the base 110 may be formed in a plate shape having a width.

The reservoir 120 may be disposed on the base 110. The accommodation space 121 in which a culture medium is accommodated may be provided inside the reservoir 120, and the through hole 123 through which the culture medium flows from the outside may be formed in one surface of the accommodation space 121.

In an embodiment, two reservoirs 120 may be disposed.

For example, the reservoirs 120 may include a first reservoir 120a and a second reservoir 120b. The first reservoir 120a may be disposed on one surface of the base 110 to be biased to one side, and the second reservoir 120b may be disposed on the one surface of the base 110 similarly to the first reservoir 120a to be spaced apart from the first reservoir 120a. Here, the first reservoir 120a may be a means for supplying a culture medium, and the second reservoir 120b may be a means for collecting the culture medium. For example, the culture medium may be supplied from the first reservoir 120a, and collected in the second reservoir 120b after undergoing material exchange with the culture as it passes through the plate unit 200.

In an optional embodiment, the first reservoir 120a and the second reservoir 120b may be formed in the same shape. In this case, based on an experimenter's choice, the first reservoir 120a may serve as a means for supplying a culture medium, and the second reservoir 120b may serve as a means for collecting the culture medium. On the contrary, based on an experimenter's choice, the first reservoir 120a may serve as a means for collecting the culture medium, and the second reservoir 120b may serve as a means for collecting the culture medium. Alternatively, the first reservoir 120a and the second reservoir 120b may alternately perform the supply and collection of the culture medium. This may depend on the experimenter's choice, and for example, as will be described below, the reservoir 120 in which a relatively large amount of culture medium is accommodated may supply the culture medium and the reservoir 120 in which a relatively small amount of culture medium is accommodated may collect the culture medium.

Hereinafter, for convenience of description, unless otherwise stated, a case in which a culture medium is supplied from the first reservoir 120a and collected in the second reservoir 120b will be described as an example.

The accommodation space 121 provided inside the reservoir 120 may be a space in which a culture medium is accommodated. For example, a culture medium necessary for culturing cultures may be accommodated in the accommodation space 121 of the first reservoir 120a, and following a set flow, the culture medium may be supplied to the plate unit 200 from the accommodation space 121. Thereafter, the culture medium that has passed through the plate unit 200 may be collected in the accommodation space 121 of the second reservoir 120b.

The through hole 123 through which a culture medium flows from the outside may be formed in one surface of the accommodation space 121, and for example, the through hole 123 may be formed to pass through a bottom surface of the accommodation space 121. In this case, the through hole 123 may be formed in each of the first reservoir 120a and the second reservoir 120b. The through hole 123 formed in the first reservoir 120a may be a path through which the culture medium is supplied from the first reservoir 120a to the plate unit 200. In addition, the through hole 123 formed in the second reservoir 120b may serve as a path through which the culture medium is collected in the second reservoir 120b from the plate unit 200.

In an embodiment, the reservoir 120 may include at least one partition wall 122 configured to partition the accommodation space 121 into a plurality of spaces. Referring to FIG. 3 again, the accommodation space 121 provided inside the reservoir 120 may be partitioned by the partition wall 122, and for example, the reservoir 120 may include a plurality of partition walls 122 allowing the accommodation space 121 to be partitioned into a plurality of spaces. Here, the plurality of partitioned spaces may be isolated from each other to prevent mixing of culture media. Thus, various culture media or various drugs can be supplied to the cultures.

In this case, the through hole 123 may be formed in each of the plurality of spaces partitioned by the partition walls 122. Thus, the culture media may be individually supplied from each space to the plate unit 200.

In an optional embodiment, referring to FIG. 2 again, the cell culture device 10 according to an embodiment of the present disclosure may further include the cover 130. The cover 130 may be a means for covering an open portion of the reservoir 120 to prevent foreign substances from entering the culture medium from the outside.

Accordingly, the cover 130 may be removed to open the reservoir 120 during the filling of the culture medium in the reservoir 120, and may be disposed at the open position of the reservoir 120 to close the reservoir 120 during the culturing process.

The cover 130 may cover at least one of the first reservoir 120a and the second reservoir 120b, and preferably, two covers 130 may be provided to cover both the first reservoir 120a and the second reservoir 120b.

The culture medium supply unit 100 may include the culture medium control member 140 configured to control the flow of the culture medium discharged from the reservoir 120. For example, the culture medium control member 140 may control a flow rate and a flow volume of the culture medium discharged from the reservoir 120.

In an embodiment, the culture medium control member 140 may be formed to have a length, and the culture medium control member 140 may include a porous material.

The culture medium control member 140 may include a porous material to create resistance to the flow of the culture medium. For example, the culture medium control member 140 may be disposed to pass through the through hole 123 of the reservoir 120, and accordingly, the culture medium may move to the plate unit 200 through the culture medium control member 140. Here, since the culture medium control member 140 includes a porous material, resistance to the flow of the culture medium may be created so that the flow rate and volume of the culture medium are controlled accordingly.

The culture medium control member 140 may include various materials according to the resistance to the flow of the culture medium. For example, when a particular culture medium control member 140 is disposed in the through hole 123 of the reservoir 120, the flow of the culture medium can be controlled to match a resistance value generated by the corresponding culture medium control member 140. Accordingly, various types of culture medium control members 140 may be provided so that the culture medium can be provided at various flow rates or volumes according to specific situations, such as the goals, methods, or the like of the culturing.

In an optional embodiment, the culture medium control member 140 may be detachable, and for example, the culture medium control member 140 may be replaceable. Specifically, when an experimenter desires a specific flow rate or flow volume for a culture medium for the experiment, the experimenter may select the culture medium control member 140 capable of generating a flow corresponding to the specific flow rate or volume and install the culture medium control member 140 in the reservoir 120.

In an optional embodiment, the reservoir 120 may be formed such that a bottom surface of the accommodation space 121 in which the culture medium is accommodated is spaced apart from the base 110 by a predetermined distance. For example, as shown in FIG. 4, a perimeter of the reservoir 120 may be formed to extend such that the bottom surface is spaced apart from the base 110. In addition, the through hole 123 may be formed in the bottom surface of the accommodation space 121, and the through hole 123 may be formed to extend from the bottom surface of the accommodation space 121 in a direction toward the base 110.

In addition, the base 110 may include a protrusion that is formed to extend from the base 110 in a direction toward the accommodation space 121 at a position corresponding to the through hole 123 formed in the accommodation space 121, and has a through groove formed therein. One end of the through groove formed in the protrusion may be open in a direction toward the reservoir 120, and the other end thereof may be open in a direction toward the plate unit 200. Thus, the through hole 123 formed in the reservoir 120 and the protrusion formed on the base 110 are formed to face each other, and a space through which the culture medium may flow from the accommodation space 121 of the reservoir 120 to the plate unit 200 may be provided. However, the through hole 123 and the protrusion are not necessarily in contact with or spaced apart from each other.

Here, the culture medium control member 140 may be formed to have a length, and may be disposed to pass through the through hole 123 formed in the reservoir 120 and the protrusion formed on the base 110. Accordingly, the culture medium stored in the accommodation space 121 may be absorbed by the culture medium control member 140 and moved along the culture medium control member 140. That is, the culture medium may be supplied to the plate unit 200 from the accommodation space 121 along the culture medium control member 140 via the through hole 123 and the through groove formed in the protrusion. During this process, the flow of the culture medium is subjected to resistance, so the flow rate or volume may be controlled.

The culture medium control member 140 is preferably provided in the reservoir 120 that supplies the culture medium, but may not be provided in the reservoir 120 that collects the culture medium.

For example, when the experimenter wants to always generate the flow of the culture medium only in one direction, the culture medium control member 140 may be installed only in the reservoir 120 that supplies the culture medium.

Alternatively, when the experimenter wants to control a flow direction of the culture medium as needed, the culture medium control member 140 may be installed in both the reservoir 120 that supplies the culture medium and the reservoir 120 that collects the culture medium.

As described above, by including the culture medium control member 140 having resistance to the flow of the culture media, the cell culture device 10 according to the present embodiment can prevent cells present in a well 230 from being swept away due to the culture media flowing at an excessively high speed.

In addition, the culture medium control member 140 may be adjusted so that the flow rate or volume of the culture medium is similar to that of the microenvironment of the human body.

In addition, the culture medium control member 140 may control the flow rate or volume of the culture medium as needed to facilitate analysis of the effects on the cultures while varying the flow rate or volume of the culture medium.

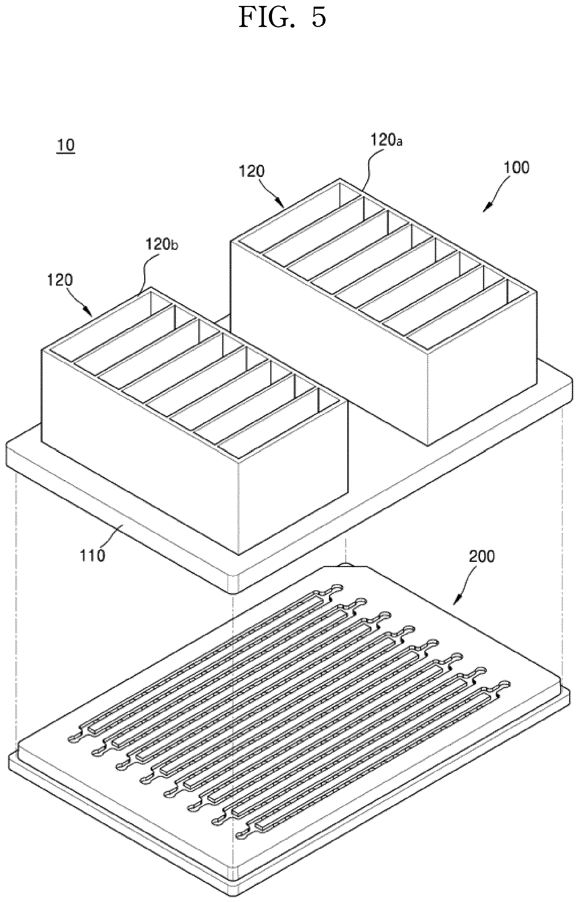

FIG. 5 is a view for describing a usage example of the cell culture device 10 according to an embodiment of the present disclosure.

Referring to FIG. 5, the plate unit 200 may be detachable from the culture medium supply unit 100.

In an embodiment, the cell culture device 10 according to an embodiment of the present disclosure may be used in a coupled state only during the cultivation of cultures or when experiments are being conducted on the cultures Specifically, the culture medium supply unit 100 and the plate unit 200 may be manufactured, stored, repaired, and maintained in a state of being separated from each other, and may be coupled to each other and used when in use. Thus, maintenance and repair may be facilitated.

In an optional embodiment, based on FIG. 5, a stepped portion may be formed along a perimeter of the plate unit 200, and specifically, the stepped portion may be formed so that a surface of the plate unit 200 in contact with the floor has an area greater than that of an upper layer thereabove. In addition, the culture medium supply unit 100 may have a bottom surface formed in a shape indented toward the inside thereof, allowing the stepped portion formed in the plate unit 200 to be inserted into the bottom surface.

Accordingly, the plate unit 200 may be easily inserted into and coupled to the bottom surface of the culture medium supply unit 100. In addition, with such coupling, the plate unit 200 and the culture medium supply unit 100 may remain aligned without any wobbling or deviation in the coupled state.

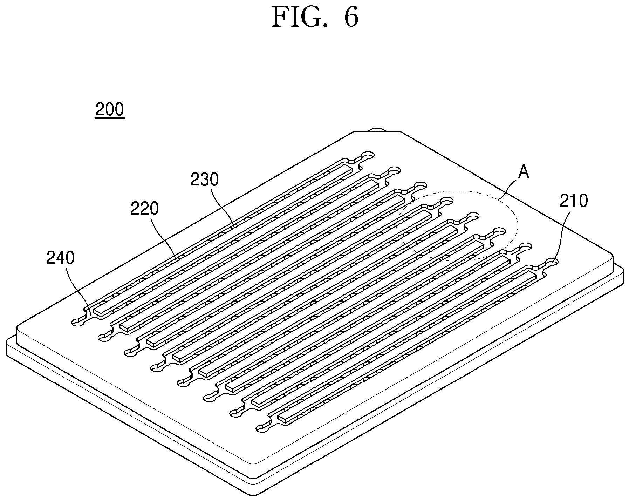

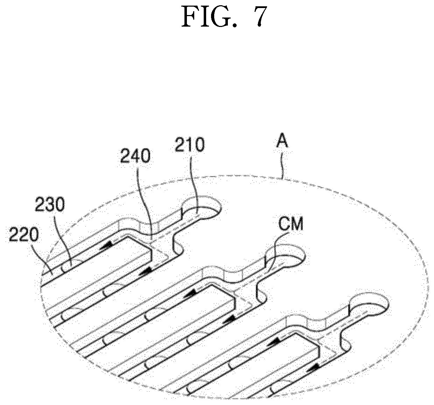

FIG. 6 is a view schematically illustrating the plate unit 200 of FIG. 1, and FIG. 7 is an enlarged view of portion A of FIG. 6.

Referring to FIGS. 6 and 7, the plate unit 200 may be a means for receiving a culture medium CM from the reservoir 120 and providing a path through which the supplied culture medium CM may flow.

The plate unit 200 may include a channel 220, which guides a path of the culture medium CM supplied from the culture medium CM supply unit 100, and at least one well 230 disposed to be connected to the channel 220.

The plate unit 200 may include a collection part 210 to which the culture medium CM is first supplied from the reservoir 120. The collection part 210 is disposed below the culture medium CM control member 140, and the culture medium CM that has moved along the culture medium CM control member 140 may be supplied to the collection part 210.

The culture medium CM may move along the channel 220 from the collection part 210. The channel 220 may provide a moving path for the culture medium CM.

In an embodiment, the collection part 210 may be disposed on each of both ends of the channel 220. Here, the collection part 210 disposed on one end of the channel 220 may be a part in which the culture medium CM, which is being supplied, is collected, and the collection part 210 disposed on the other side of the channel 220 may be a part in which the culture medium CM, which is collected by moving through the channel 220, is collected.

A plurality of channels 220 may be disposed, and the culture medium CM may move along the plurality of channels 220.

When the accommodation space 121 of the reservoir 120 is partitioned into a plurality of spaces as described above, the channels 220 may be disposed to correspond to the plurality of partitioned spaces. For example, when the accommodation space 121 is partitioned into a plurality of spaces and multiple types of culture media CM are supplied, the culture media CM may not be mixed with each other by respectively moving along different channels 220.

In an optional embodiment, the plate unit 200 may further include a branch part 240 disposed between the collection part 210 and the channels 220. The branch part 240 may be a part that branches off from the collection part 210 into multiple sections, each of which is connected to the respective channels 220. Accordingly, a plurality of channels 220 may be disposed in parallel between the collection parts 210 that are disposed on both sides of the plate unit 200.

Thus, the same culture medium CM can flow along the plurality of channels 220, enabling the cultivation of a large amount of cultures or facilitating observation of changes caused by supplying the same culture medium CM to the different cultures.

As such, the plate unit 200 may include the plurality of channels 220 disposed in parallel, and the same drug or different drugs may be supplied to the plurality of channels 220 according to the experimenter's choice. Accordingly, the cell culture device 10 according to the present disclosure can add various drugs such as various anti-cancer agents and promoters to the culture medium, thereby enabling various drug evaluations to be performed on the cultures.

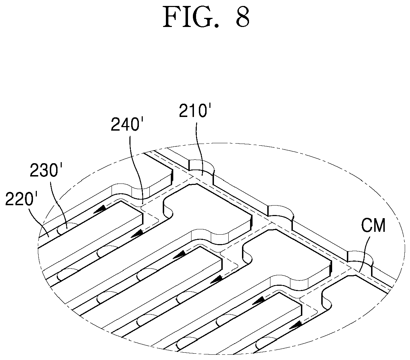

FIG. 8 is a view schematically illustrating one modified example of a plate unit 200'.

Referring to FIG. 8, a plurality of collection parts 210' may be formed to be connected to each other.

In this case, a culture medium CM supplied to one collection part 210' may move to the adjacent collection parts 210'. Accordingly, the present modified example may be used when the same culture medium CM is to be simultaneously supplied to a plurality of cultures.

However, although a case in which the plurality of collection parts 210' are connected to each other is illustrated in the drawing as an example, the collection parts 210' are not limited thereto and may be connected such that channels 220' or branch parts 240' communicate with each other.

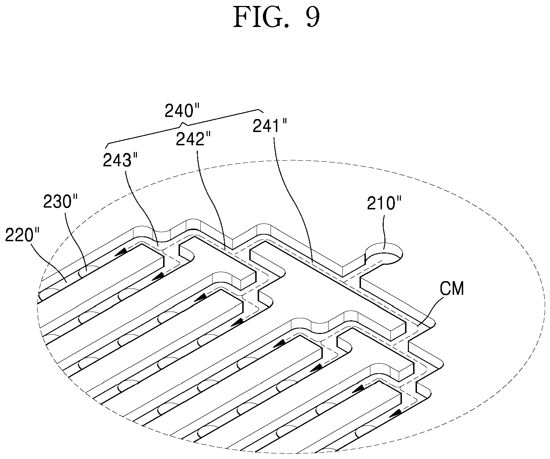

FIG. 9 is a view schematically illustrating another modified example of a plate unit 200".

Referring to FIG. 9, a culture medium CM collected in one collection part 210" of the plate unit 200" may be branched by a branch part 240" and supplied to a plurality of channels 220". In this case, the branch part 240" may include at least one segmented branch part.

Specifically, the branch part 240" may include a first branch part 241", a second branch part 242", and a third branch part 243". Here, the culture medium CM supplied to the collection part 210" may be branched by the first branch part 241", and the branched culture medium CM may be branched again at the second branch part 242" and then branched again at the third branch part 243" to flow into the channel 220". That is, the culture medium CM supplied to one collection part 210" may be branched into a plurality of sections while passing through the plurality of segmented branch parts 241 ", 242", and 243" and then supplied to the channels 220", and thus the same culture medium CM may be supplied to a plurality of wells 230".

Accordingly, even when a large amount of culture medium CM is supplied to the collection part 210", the supplied culture medium CM is branched into a plurality of sections and supplied to the channel 220", so that a flow rate and a flow volume thereof can be relatively reduced. That is, the flow rate and volume of the supplied culture medium CM can be easily controlled according to the experimenter's choice.

In addition, by supplying the culture medium CM to only one collection part 210", the same culture medium CM can be simultaneously supplied to the plurality of channels 220", so that culturing and drug evaluation can be performed more easily and efficiently.

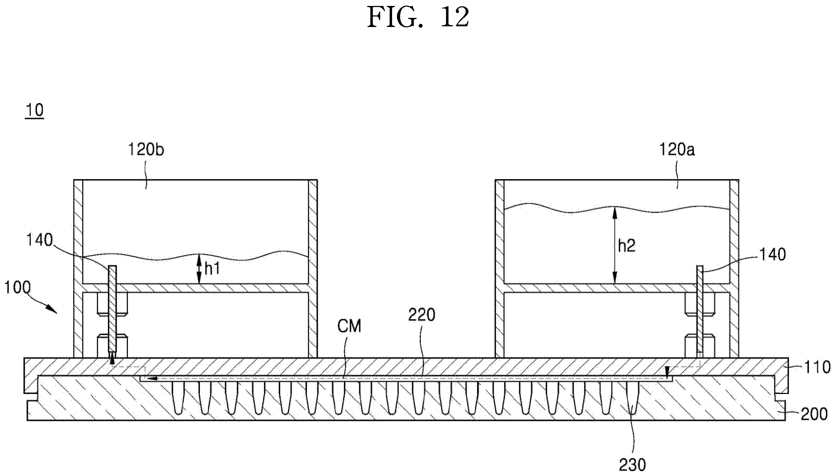

However, referring to FIG. 12, in the present embodiment, the first branch part 241 ", the second branch part 242", and the third branch part 243" are included, but the present invention is not limited thereto. and only two segmented branch parts or three or more segmented branch parts may be included as needed.

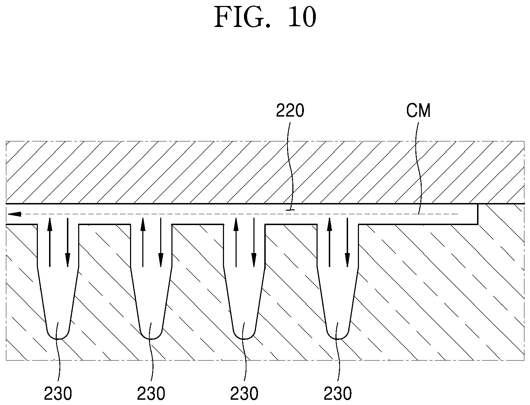

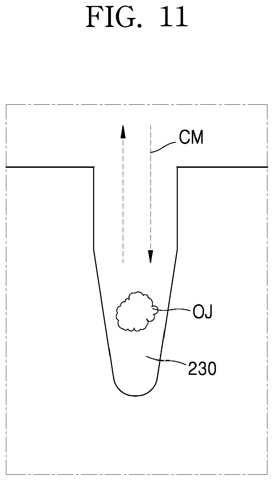

FIG. 10 is a view for describing the flow of the culture medium CM, and FIG. 11 is a view for describing a process of supplying the culture medium CM to a culture OJ.

Referring to FIGS. 10 and 11, the plate unit 200 may include the well 230.

The well 230 may be a part in which the culture OJ is disposed. For example, a space in which the culture OJ is accommodated may be provided inside the well 230.

In an embodiment, the well 230 may be disposed on a path through which the culture medium CM flows, and for example, the well 230 may be disposed to be connected to the channel 220.

Thus, the culture medium CM may be supplied to the culture OJ while flowing along the channel 220. Here, the culture medium CM may be supplied to the culture OJ by diffusion.

Specifically, while the culture medium CM flows along the channel 220, nutrients, oxygen, and other additives contained in the culture medium CM may be supplied to the culture OJ by diffusion. In addition, when a drug is contained in the culture medium CM, the drug may be supplied to the culture OJ. Furthermore, metabolites of the culture OJ may diffuse into and enter the culture medium CM flowing along the channel 220. Thus, the culture medium CM flowing along the channel 220 facilitates easy substance exchange within the culture OJ.

In an embodiment, the wells 230 are disposed along a length of the channel 220, and may be disposed below the channel 220. Thus, the culture medium CM can be more easily supplied to the culture OJ disposed inside the well 230.

In an embodiment, a plurality of wells 230 may be disposed along the length of the channel 220. That is, since the channel 220 provides a flow path for the culture medium CM in a length direction, the plurality of wells 230 can be disposed in the flow direction of the culture medium CM.

Thus, a plurality of wells 230 may be connected to each channel 220, thereby enabling simultaneous culturing of a large amount of culture OJ.

FIG. 12 is a view for describing the principle in which the culture medium CM flows.

Referring to FIG. 12, the culture medium CM may flow from the first reservoir 120a to the second reservoir 120b. At this time, depending on a height of the culture medium CM filled in each reservoir 120, the reservoir 120 may be classified into the reservoir 120 that supplies the culture medium CM and the reservoir 120 that collects the culture medium CM.

Specifically, a direction in which the culture medium CM flows may be controlled according to a relative size of the height of the culture medium CM filled in each of the first reservoir 120a and the second reservoir 120b. For example, based on FIG. 12, the first reservoir 120a is the reservoir 120 disposed on the right side, the second reservoir 120b is the reservoir 120 disposed on the left side, and in this case, a height h2 of the culture medium CM filled in the first reservoir 120a may be greater than a height h1 of the culture medium CM filled in the second reservoir 120b. Accordingly, the culture medium CM stored in the first reservoir 120a may be supplied to the plate unit 200 by gravity, and the culture medium CM may flow along the channel 220 and then be collected in the second reservoir 120b.

On the contrary, when the height h1 of the culture medium CM filled in the first reservoir 120a is greater than the height h2 of the culture medium CM filled in the second reservoir 120b, the flow may be reversed. That is, the culture medium CM may flow from the first reservoir 120a to the second reservoir 120b.

Further, when the height h1 of the culture medium CM filled in the first reservoir 120a is equal to the height h2 of the culture medium CM filled in the second reservoir 120b, the flow of the culture medium CM may stop.

As described above, when the cell culture device 10 according to the present disclosure is used, three-dimensional cell spheroids and organoids can be cultured in an environment similar to the microenvironment of the human body. In addition, in performing drug evaluation on the three-dimensional cell spheroids and organoids, the drug evaluation can also be performed in an environment similar to the microenvironment of the human body.

Specifically, in the cell culture device 10 according to the present disclosure, it is possible to form the culture OJ in large quantities and promote uniform formation of the culture OJ to a certain size, so that standardization of the culture OJ may become possible. In addition, according to the present disclosure, drug evaluation using various drugs such as anti-cancer agents can be simultaneously performed on a plurality of cells and can be screened, so that uniform drug evaluation results can be derived, and the present disclosure can be used to extract multiple samples or provide patient-customized precision medicine.

The present disclosure has been described with reference to the embodiments illustrated in the drawings, but these are only examples. It will be understood by those skilled in the art that various modifications and equivalent other embodiments may be made. Accordingly, the true technical scope of the present disclosure is defined by the technical spirit of the appended claims.

The particular implementations shown and described herein are illustrative examples of the embodiments and are not intended to otherwise limit the scope of the embodiments in any way. For the sake of brevity, conventional electronics, control systems, software development and other functional aspects of the systems may not be described in detail. Further, the connecting lines or connectors shown in the drawings are intended to represent example functional relationships and/or physical or logical couplings between the various elements. It should be noted that many alternative or additional functional relationships, physical connections, or logical connections may be present in a practical device. In addition, no item or component is essential to the practice of the present disclosure unless the component is specifically described as "essential" or "critical."

The use of the terms "a" and "an" and "the" and similar referents in the context of describing the present disclosure (especially in the context of the following claims) are to be construed to cover both the singular and the plural. Further, recitation of ranges of values herein is merely intended to serve as a shorthand method of referring individually to each separate value falling within the range, unless otherwise indicated herein, and each separate value is incorporated into the specification as if it were individually recited herein. Finally, operations of all methods described herein can be performed in any suitable order unless otherwise indicated herein or otherwise clearly contradicted by context. The present disclosure is not necessarily limited to the described order of the operations. The use of any and all examples, or exemplary terms (e.g., "such as") provided herein, is intended merely to better illuminate the present disclosure and does not pose a limitation on the scope of the present disclosure unless otherwise claimed. Also, numerous modifications and adaptations will be readily apparent to one of ordinary skill in the art without departing from the spirit and scope of the present disclosure.

### INDUSTRIAL APPLICABILITY

According to an embodiment of the present disclosure, provided is a cell culture device capable of efficiently performing large-scale and long-term culturing and drug evaluation of cultures such as three-dimensional cell spheroids or organoids. In addition, the embodiment of the present disclosure may be applied to a cell culture device for industrial use.

## Claims

1. A cell culture device comprising a culture medium supply unit including:
a base;
a first reservoir disposed on the base and having an accommodation space, which accommodates the culture medium and has one surface in which a through hole through which the culture medium is discharged is formed; and
a culture medium control member disposed to pass through the through hole and configured to control a flow of the culture medium discharged from the first reservoir.

2. The cell culture device of claim 1, wherein the culture medium supply unit further includes a second reservoir disposed on the base to be spaced apart from the first reservoir.

3. The cell culture device of claim 1, wherein the reservoir includes at least one partition wall configured to partition the accommodation space into a plurality of spaces.

4. The cell culture device of claim 3, wherein, in the reservoir, a through hole is formed in each of the plurality of spaces partitioned by the at least one partition wall.

5. The cell culture device of claim 1, wherein the culture medium control member is made of a porous material.

6. The cell culture device of claim 1, further comprising a plate unit including:
a channel configured to guide a path through which the culture medium is supplied from the culture medium supply unit; and
at least one well disposed to be connected to the channel.

7. The cell culture device of claim 6, wherein the plate unit is detachable from the culture medium supply unit.

8. The cell culture device of claim 6, wherein the channel is provided in plurality.

9. The cell culture device of claim 6, wherein the at least one well is disposed in plurality along a length of the channel.
